# EUROPEAN PATENT APPLICATION

(11) **EP 2 908 112 A1**
(43) Date of publication of application: **19.08.2015**
(21) Application number: 13845998.7
(22) Date of filing: 12.09.2013
(51) Int. Cl.: G01L 25/00, A61M 25/092

(54) **MEASUREMENT INSTRUMENT CALIBRATION DEVICE AND CALIBRATION METHOD**

(30) Priority: 10.10.2012 JP 2012225113
(71) Applicant: NTN Corporation, Osaka-shi, Osaka 550-0003 (JP); National University Corporation Nagoya University, Aichi 464-8601 (JP)
(72) Inventor: YAMADA, Hiroyuki, Iwata-shi Shizuoka 438-8510 (JP); MARUI, Naoki, Iwata-shi Shizuoka 438-8510 (JP); MIYACHI, Shigeru, Nagoya-shi Aichi 464-8601 (JP); MATSUBARA, Noriaki, Nagoya-shi Aichi 464-8601 (JP)
(74) Representative: Bockhorni & Kollegen
(86) International application number: PCT/JP2013/074672
(87) International publication number: WO 2014/057763

(57) **Abstract**

It is a calibration device for a measurement instrument (101) for measuring a compressive force or a tensile force in a longitudinal direction exerted to a linear body (11) from a predetermined correlation. A stopper part (104) for fixing the linear body (11) is fixed at a one end side entrance (3) and a push/pull movable part (105) for enabling the linear body (11) to be pushed and pulled in an insertion/withdrawal direction at an other end entrance (4) is fixed, so that the relative positions of the stopper part and the push/pull movable part with respect to a sensor main body (2) cannot be changed. A force detector (109) measures the force in the insertion/withdrawal direction exerted from the push/pull movable part (105) to the linear body (11). The force detector (109) is interposed between the push/pull movable part (105) and the linear body (11).

## Description

### TECHNICAL FIELD

The present invention relates to a calibration device and a calibration method for a measurement instrument, and more particularly to a calibration device and a calibration method for a measurement instrument which is configured to measure an operating force on a wire or the like used in a catheter treatment.

### BACKGROUND ART

As a medical equipment of a body-insertion type, there has been a known linear body such as a guide wire or a catheter inserted into a vessel such as a blood vessel or a urinary duct.

Moreover, there has been a known wire provided with an embolus coil at a leading end thereof to embolize an aneurysm.

Such a thin wire-shaped object is inserted into a vessel of a human body, operated from outside of the human body, and guided to a target site.

A vessel in a human body is not straight but has curves and branches. Therefore, a skill is required for the guiding operation from outside.

Particularly, when an excessive load is exerted to a vessel in a body by a thin wire during the operation, there is a risk of damaging the vessel.

For example, Japanese Patent Laying-Open No. 10-263089 (PTD 1) discloses a catheter having an obstacle sensing function, wherein a pressure sensor is provided at a leading end of a guide wire to serve as a device for preventing damage to a vessel in a body.

Moreover, there has been a known compressive force measurement instrument for a linear body, wherein a sensor for detecting the curvature of the linear body is used to prevent damage to a vessel in a body.

For example, Japanese Patent Laying-Open No. 2008-064508 (PTD 2) discloses a compressive force measurement instrument for measuring a compressive force of a linear body.

### CITATOIN LIST

### PATENT DOCUMENT

PTD 1: Japanese Patent Laying-Open No. 10-263089
PTD 2: Japanese Patent Laying-Open No. 2008-064508

### SUMMARY OF INVENTION

### TECHNICAL PROBLEM

However, in the case of a guide wire, particularly in the case of a guide wire inserted into a brain blood vessel as with PTD 1, a diameter of a leading end of the guide wire may have a thickness of about 0.35 mm. It is difficult to attach a small-sized pressure sensor at a leading end of such an extra-thin guide wire.

Moreover, it is necessary to have wiring pass through a guide wire to take out a signal of the pressure sensor to outside of a human body. However, this operation further requires a difficulty.

Therefore, as shown in PTD 2, there has been a known optical position sensor for detecting the curvature of a linear body by a position. By detecting a position of the guide wire with use of this position sensor, a compressive force can be measured in place of the pressure sensor provided at a leading end.

Specifically, a cavity is formed in a sensor main body of the position sensor to penetrate therethrough, wherein the cavity allows insertion of a guide wire as a linear body in the state where the guide wire is curved at a desired angle.

When a tensile force or a compressive force is exerted to the sensor main body, a position of the linear body is displaced inward and outward in a radial direction of an arc in accordance with a change in the curvature. This displacement is detected by the position sensor, so that the compressive force and the tensile force are measured.

Further, the optical sensor used as the position sensor includes a light emitting unit irradiating light in the cavity and a light receiving unit including a plurality of light receiving elements for receiving light.

The optical sensor selects a plurality of light receiving elements from the light receiving elements in the light receiving unit based on a quantity of light received by the light receiving elements in the light receiving unit, and performs a predetermined calculation with use of the quantity of light received by the plurality of selected light receiving elements.

This optical sensor is connected to a measurement instrument control unit for detecting a position of the linear body.

The measurement instrument control unit performs a calculation of a compressive force exerted to the linear body in accordance with a conversion from the detected position of the linear body.

When such a sensor is used in a coil embolism treatment for a cerebral aneurysm or the like, it is preferable that the sensor main body to which dirt adhere is disposable in view of safety and sanitation.

In such case, if the sensor main body is separated from the light emitting unit and light receiving unit, and the sensor main body is disposed, and the light emitting unit and light receiving unit are reusable, the cost can be suppressed. Therefore, it would be more preferable.

However, if the reusable parts are separable, the operation of assembling the optical sensor to the main body must be performed every time before the treatment.

Therefore, there has been a possibility that the positions of the sensor main body and the optical sensor are displaced due to the influence of vibration during disassembled conveyance or an assembling error during the assembling operation so that the correlation between an output of the sensor and a compressive force is changed.

Further, there has been a problem that dirt and scars may occur in the optical system during the sterilizing step or the assembling operation so that a measured value is affected.

For example, as a matter of fact, it is impossible to bring a lot of measurement instruments and calibration devices to a place such as an operating room where a strict management on sanitation in a use environment is required, to perform assembling, and to calibrate the measurement instrument every time before use.

If the assembled measurement instrument is used without calibration, it becomes unclear whether a correct measurement is performed, and there is no means to confirm the measured value.

An object of the present invention is to provide a calibration device and a calibration method for a measurement instrument which can perform calibration for the measurement instrument in a convenient manner and achieve a highly accurate measurement after the assembling operation without restriction by the use environment.

### SOLUTION TO PROBLEM

The present invention is a calibration device for a measurement instrument including a sensor main body provided with a one end side entrance and an other end side entrance which a linear body having a flexibility is inserted to and withdrawn from, a detection part allowing a displacement of the linear body, which is in communication with the sensor body, in a predetermined direction, and a position sensor provided detachably in the detection part and configured to detect a position of the linear body.

The measurement instrument measures a compressive force or a tensile force exerted in a longitudinal axis direction of the linear body from a predetermined correlation based on a position of the linear body detected by the position sensor.

The calibration device for a measurement instrument includes a stopper part for fixing one end side of the linear body at the one end side entrance, a push/pull movable part configured to locate a front side on a side opposite to the one end side to be pushed and pulled in an insertion/withdrawal direction at the other end side entrance, fixing means which fixes the stopper part and the push/pull movable part so that relative positions thereof with respect to the sensor main body cannot be changed, and a force detector interposed between the push/pull movable part and the linear body and configured to measure a force in an insertion/withdrawal direction exerted from the push/pull movable part to the linear body.

Preferably, the fixing means includes a base, and the base is provided with a measurement instrument fixing part for fixing the sensor main body on a plane on which the push/pull movable part and the stopper part are fixed, and the sensor main body, the push/pull movable part, and the stopper part are arranged at desired positions on the base.

More preferably, the fixing means includes a housing formed integrally with the sensor main body. The stopper part is mounted and fixed near a one end side entrance the housing. The push/pull movable part is mounted and fixed near an other end side entrance of the housing.

More preferably, by fixing the stopper part to the one end side entrance and fixing the push/pull movable part to the other end side entrance, the fixing means is configured to adjoin the stopper part and the push/pull movable part and to fix the stopper part and the push/pull movable part integrally with the sensor main body so as to clamp the sensor main body.

More preferably, the push/pull movable part is a linear motion guide mechanism having a movable pillar part which is slidable along an inner side of a cylindrical movable guide part.

More preferably, the push/pull movable part has a movable top member coupling a front side of the linear body and a screw member capable of adjusting a position of the movable top member.

More preferably, the push/pull movable part has a movable top member coupling a front side of the linear body and a back pressure chamber into which fluid used for adjusting a position of the movable top member is pressed.

More preferably, the stopper part has a press-fit fixing member. In a state where one end side of the linear body is inserted to a fitting hole formed in a deformable elastic member, the press-fit fixing member gives a pressure to the elastic member and deforms the same to allow an inner surface of the fitting hole to be press-fitted to a peripheral surface of the linear body.

More preferably, the stopper part has a fixing member having a recessed receiving part in which one end of the linear body is inserted and fitted.

More preferably, the stopper part has a clamping fixing member which clamps and fixes one end side of the linear body between a pair of deformable elastic members.

More preferably, the stopper part has a fitting fixing member in which one end side of the linear body is inserted and fitted to a fitting hole formed in the deformable elastic member.

More preferably, the calibration device is used for calibrating a measurement instrument incorporated in a medical equipment.

More preferably, the calibration device is used for calibrating of a measurement instrument incorporated in a training simulator.

A method for calibrating a measurement instrument according to one aspect of the present invention is a method for calibrating a measurement instrument for measuring a compressive force or a tensile force in a longitudinal axis direction exerted to a linear body having a flexibility based on a change in a curvature of the linear body. The method includes the step of inserting the linear body into a cavity of a detection part provided in the measurement instrument, the step of detecting a curvature of the linear body in the detection part, the step of detecting a compressive force or a tensile force in the longitudinal direction exerted to the linear body, and the step of carrying out calibration by comparing the detected compressive force or tensile force with a curvature of the linear body.

### ADVANTAGEOUS EFFECTS OF INVENTION

According to the present invention, the sensor main body having the detection part assembled thereto is fixed to the push/pull movable part and the fixing stopper part so that the sensor main body cannot be moved. The push/pull movable part enables the linear body to be pushed and pulled in the insertion/withdrawal direction.

When assembling a position sensor which is reusable in an operating room or the like is assembled to a new sensor main body, the force detector detects a force in the insertion/withdrawal direction exerted to the linear body.

The detected force in the insertion/withdrawal direction is compared with a position of the linear body detected by the position sensor, and can be used for calibration.

Therefore, the calibration for the measurement instrument can be performed conveniently without limitation by the use environment, and a highly accurate measurement by means of the measurement instrument can be performed.

### BRIEF DESCRIPTION OF DRAWINGS

Fig. 1 is a plan view representing a state where a measurement instrument is mounted to a calibration device in accordance with a first embodiment of the present invention.
Fig. 2 is an exterior view representing a configuration of a sensor main body of the measurement instrument in accordance with the first embodiment.
Fig. 3 is a cross-sectional view representing a cross section taken along the III-III line of Fig. 2.
Fig. 4 is an exterior perspective view representing an entire calibration device viewed from a cross section taken along the IV-IV line of Fig. 2 and a schematic block diagram representing a circuit configuration of the calibration device.
Fig. 5 is a cross-sectional view representing a cross section taken along the V-V line of Fig. 1.
Fig. 6 is a diagram representing the part which is the same as the cross-sectional view of Fig. 3 and representing a state where a linear body changes a curvature in accordance with a compressive force or a tensile force exerted in the longitudinal axis direction of the linear body inserted to the sensor main body.
Fig. 7 is a flowchart for explanation of a flow of calibration of the calibration device for the measurement instrument in accordance with the first embodiment.
Fig. 8 is a graph plotting measured values of positions due to displacement of the linear body in a state where the compressive force or the tensile force as the exerted force is exerted to the linear body.
Fig. 9 is a perspective view representing a push/pull movable part in accordance with Modified Example 1 of the embodiment.
Fig. 10 is a perspective view representing a push/pull movable part in accordance with Modified Example 2.
Fig. 11 is a plan view representing a configuration of a calibration device for a measurement instrument in accordance with a second embodiment.
Fig. 12 is a plan view representing a configuration of a calibration device for a measurement instrument in accordance with a third embodiment.
Fig. 13 is a cross-sectional view representing a push/pull movable part of Modified Example 3.
Fig. 14 is a cross-sectional view representing a push/pull movable part of Modified Example 4.
Fig. 15 is a cross-sectional view representing a stopper part of Modified Example 5.
Fig. 16 is a cross-sectional view representing a stopper part of Modified Example 6.
Fig. 17 is a cross-sectional view representing a stopper part of Modified Example 7.
Fig. 18 is a cross-sectional view representing a stopper part of Modified Example 8.
Fig. 19 represents a state where a calibrated measurement instrument is used in a fourth embodiment.
Fig. 20 represents a state where a calibrated measurement instrument is used for a simulation device in a fifth embodiment.

### DESCRIPTION OF EMBODIMENTS

Hereinafter, embodiments of the present invention will be described with reference to the drawings. In the following description, the same parts have the same reference numerals allotted. The names and functions of those are also the same. Thus, detailed description thereof will not be repeated.

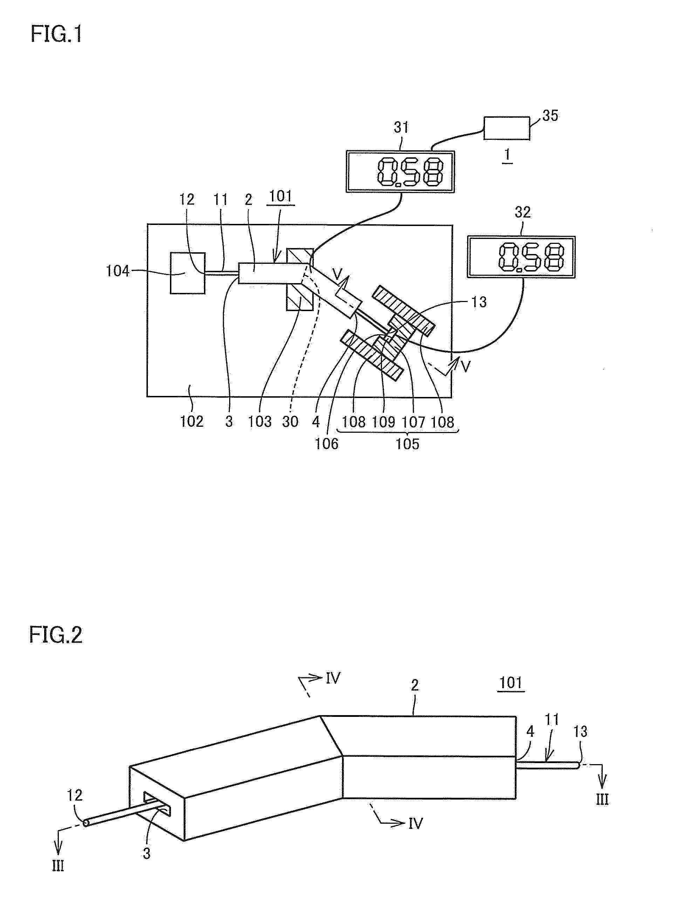

Figs. 1 to 20 represent a calibration device used for a measurement instrument 101 for measuring a compressive force and a tensile force of a linear body, and a method for calibrating measurement instrument 101 in the embodiments of the present invention.

### [First Embodiment]

Fig. 1 is a plan view representing a state where measurement instrument 101 is mounted to a calibration device 1 in accordance with a first embodiment of the present invention.

### [Overall Configuration of Calibration Device]

Referring to Fig. 1, a sensor main body 2 of measurement instrument 101 is fixed to a plate-like base 102 through a measurement instrument fixing part 103.

In sensor main body 2, a line sensor 30 as a position sensor is provided.

A sensor output indicator 31 is connected to line sensor 30.

Line sensor 30 detects a position of linear body 11 constituted of a guide wire or the like, and a compressive force or a tensile force exerted in a longitudinal axis direction of linear body 11 is measured based on a predetermined correlation from the detected position detected linear body 11.

Then, sensor output indicator 31 indicates the measured compressive force or tensile force as a numeral value visualized.

A one end side entrance 12 of linear body 11 protruding from a one end side entrance 3 of sensor main body 2 is fixed by stopper part 104 so that linear body 11 cannot be inserted or withdrawn.

Moreover, at an end opposite to one end side entrance 3 of sensor main body 2 an other end side entrance 4 is provided as an opening.

A front side end 13 of linear body 11 protruding from other end side entrance 4 is connected to a push/pull movable part 105 which can push and pull linear body 11 in an insertion/withdrawal direction.

Push/pull movable part 105 has a movable guide part 108, a movable part 107 which slides along movable guide part 108, and a force detector 109 which is mounted to a pressure-receiving recesses part 106 provided at movable part 107.

Force detector 109 is interposed between front side end 13 of linear body 11 and a bottom face of pressure-receiving recessed part 106 and detects a force in the insertion/withdrawal direction exerted to linear body 11.

A force detection indicator 32 is connected to force detector 109 and can visualize a force exerted to force detector 109 and indicate the force.

Force detector 109 is mainly constituted of a load sensor but is not particularly limited to this. For example, it may be constituted of a position sensor.

When force detector 109 is constituted of a position sensor, a force in the insertion/withdrawal direction is measured in advance, and a relationship with the amount of displacement (position) is retained. Then, the amount of displacement measured by the position sensor is converted into the force in the insertion/withdrawal direction. Accordingly, the position sensor can be used as force detector 109.

The force in the insertion/withdrawal direction measured by force detector 109 and the position of linear body 11 detected by line sensor 30 are compared, and a predetermined correlation is calibrated.

### [Detailed Configuration of Measurement Instrument]

Fig. 2 is an appearance diagram representing a housing constituting sensor main body 2 of measurement instrument 101. The exterior part of sensor main body 2 is constituted of a housing which is bent into a V-shape at a center part in a longitudinal direction.

Sensor main body 2 is a transparent body and may be formed with a substance which allows light to pass therethrough.

Moreover, sensor main body 2 has a constant thickness in a height direction and is bent into a V-shape in a top view. One end side entrance 3 is formed as an opening at one end side in the longitudinal direction of sensor main body 2, and other end side entrance 4 is formed as an opening at the other end side.

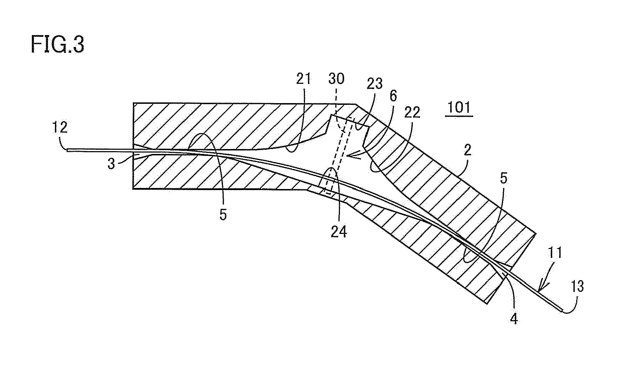

Fig. 3 is a cross-sectional view representing a cross section taken along the III-III line of Fig. 2 for explanation of an internal structure of sensor main body 2.

At a substantially center part in the longitudinal direction (near the bent part) inside sensor main body 2, a cavity-like detection part 6 is formed. Cavity-like detection part 6 communicates with one end side entrance 3 through one confining part 5 and communicates with other end side entrance 4 through another confining part 5.

Linear body 11 is inserted to sensor main body 2 from any one of one end side entrance 3 and other end side entrance 4.

In sensor main body 2, linear body 11 is slidable along the longitudinal direction, and is maintained at the position indicated by the solid line in Fig. 3 in the state of being curved at a desired angle under an unloaded condition.

When front side end 13 of linear body 11 is inserted and withdrawn through other end side entrance 4 in this state, one end side end 12 of linear body 11 is inserted and withdrawn through one end side entrance 3.

A sliding resistance generated at one end side end 12 causes the curvature in detection part 6, so that a position of linear body 11 is displaced inward or outward in the radial direction of an arc.

By detecting the position of linear body 11 at this time by the position sensor can measure a compressive force or a tensile force.

Detection part 6 includes, in the cavity formed in sensor main body 2, a line sensor 30 as a position sensor.

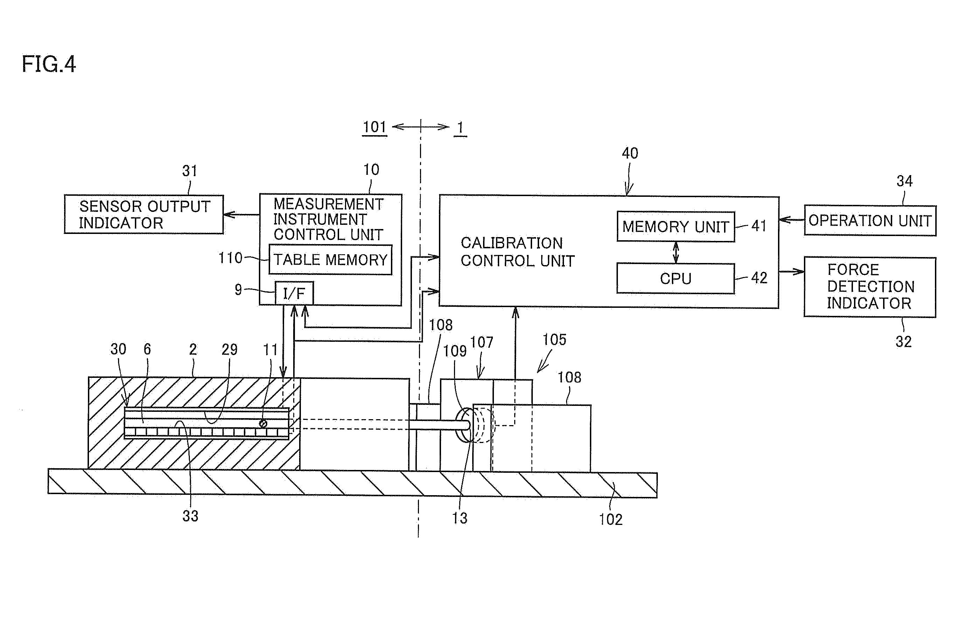

Fig. 4 is an appearance perspective view representing entire calibration device 1 viewed from the cross section taken along the IV-IV line of Fig. 2, and a circuit configuration of calibration device 1.

Referring to Fig. 4, line sensor 30 is an optical sensor and includes a light source equipment 29 as a light emitting unit emitting light in the cavity and a light receiving unit 33 including a plurality of light receiving elements receiving light.

This line sensor 30 is arranged substantially perpendicular to the longitudinal axis direction of linear body 11. Line sensor 30 is used for detecting a position of linear body 11 displaced inward and outward in the curving direction by a compressive force or a tensile force in the longitudinal axis direction exerted to linear body 11 having a flexibility.

In other words, on a side of a ceiling surface of measurement instrument 101 across linear body 11, light source equipment 29 is detachably mounted so as to face light receiving unit 33.

Referring back to Fig. 3, line sensor 30 constituted of these light source equipment 29 and light receiving unit 33 is arranged to cross inside of detection part 6 from an inner wall 24 of detection part 6 to a wall surface of a recess 23 constituting an opposite inner wall.

On opposite sides of the cavity of detection part 6, a pair of confining parts 5, 5 having an opening area with a size close to the cross section of linear body 11 are formed respectively.

Linear body 11 located in the cavity of detection part 6 is retained to curve in a predetermined arc in an unloaded state by these confining parts 5, 5.

When a compressive force or a tensile force in the longitudinal axis direction is exerted to linear body 11, linear body 11 is displaced in an arc and changes its curvature.

Line sensor 30 is arranged in the direction perpendicular to the longitudinal axis direction of linear body 11 at detection part 6. Therefore, line sensor 30 can detect a position of a top of the curvature when linear body 11 is curved to be an arc.

### [Restriction on Displacement in Cavity]

Moreover, as shown in Fig. 3, the curved shape of the surface of the inner wall in detection part 6 is configured to prevent occurrence of bending causing plastic deformation of linear body 11.

Recess 23 is formed between inner wall 21 and inner wall 22 of detection part 6. In detection part 6, recess 23 located between inner wall 21 and inner wall 22 is formed to depress toward the outer side of sensor main body 2 so that the wall surface is provided further apart from inner wall 24.

The wall part of detection part 6 is formed to have a shape of combining inner walls 21, 22, having a curved shape of the surface protruding toward an inner side of detection part 6, and recess 23.

With such a shape of detection part 6, when linear body 11 is curved by a compressive force in the longitudinal axis direction exerted to linear body 11 at detection part 6, linear body 11 can curve along the inner walls (in other words, inner wall 21 and inner wall 22) of detection part 6 located at the outer side of the curvature of linear body 11.

Moreover, a part of linear body 11 can be curved to be apart from inner wall 21 and inner wall 22. Moreover, as the compressive force increases, a distance between connection points decreases which are the points where linear body 11 extends apart from inner walls 21, 22.

Therefore, buckling of linear body 11 inside of detection part 6 can be suppressed.

In other words, even in the case where linear body 11 having a small buckling load is used, since linear body 11 curves without buckling at detection part 6, the curvature of linear body 11 can be detected in a highly accurate manner.

By converting the detected curvature into a force based on a predetermined correlation, a compressive force in the longitudinal axis direction exerted to linear body 11 can be measured.

Moreover, since recess 23 is formed at detection part 6, the compressive force exerted to linear body 11 can be measured in a highly accurate manner in a wide range. In other words, for example, a height of the curvature of linear body 11 at detection part 6 is detected. Accordingly, the compressive force exerted to linear body 11 is measured.

At this time, as long as the top of the curved part of linear body 11 in detection part 6, in other words, the point which is most apart from inner wall 24 in linear body 11 in detection part 6 is not in contact with the inner wall of detection part 6, a compressive force exerted to linear body 11 can be measured.

When recess 23 is formed, a greater compressive force in the longitudinal axis direction is required to allow the top of the curved part of linear body 11 to come into contact with the inner wall of detection part 6. Thus, the range for measuring the compressive force exerted to linear body 11 can be extended.

### [Shape Around Entrance Part]

Referring to Figs. 2 and 3, one end side entrance 3, into which one end side end 12 of linear body 11 is inserted, and other end side entrance 4, into which front side end 13 of linear body 11 is inserted so as to be insertable and withdrawable, have a tapered shape so as to improve an ability in insert linear body 11.

One end side entrance 3 and other end side entrance 4 have confining parts 5, 5 restricting the movement of linear body 11 toward the direction other than the longitudinal axis direction. At confining parts 5, 5, diameters of one end side entrance 3 and other end side entrance 4 are slightly larger than the diameter of linear body 11 (for example, 105% to 120% of the diameter of linear body 11).

Moreover, the dimension in the length direction between one end side entrance 3 and other end entrance 4 along the longitudinal axis direction of linear body 11 is set to be apart by several folds of the dimension of linear body 11 in the direction of the diameter. Thus, at confining part 5, 5, linear body 11 is freely slidable in the longitudinal axis direction, and its movement in the direction other than the longitudinal axis direction is confined.

Linear body 11 inserted to the cavity of detection part 6 from one end side entrance 3 is guided to lead out from other end entrance 4 on an opposite side through the other confining part 5. Linear body 11 inserted to the cavity of detection part 6 from other end entrance 4 through one confining part 5 is guided to lead out from one end side entrance 3 on an opposite side through the other confining part 5.

In the cavity of detection part 6 provided with confining parts 5, 5 on opposite sides, when a compressive force is exerted to linear body 11, the curvature of linear body 11 increases as compared to the case where the compressive force or tensile force is not exerted to linear body 11.

Moreover, when the tensile force is exerted to linear body 11, the curvature of linear body 11 decreases as compared to the case where the compressive force or tensile force is not exerted to linear body 11. Accordingly, the height of the curvature of linear body 11 is determined.

### [Circuit Configuration]

Fig. 4 represents an appearance perspective view of entire calibration device 1 viewed from the cross section taken along the IV-IV line of Fig. 2 and a schematic block diagram representing the circuit configuration of calibration device 1.

Referring to Fig. 4, measurement instrument 101 includes line sensor 30 constituted of light source equipment 29 for emitting light and light receiving unit 33 for receiving light emitted from light source equipment 29, and a measurement instrument control unit 10 controlling light source equipment 29 to emit light.

Light receiving unit 33 is a one-dimensional optical array sensor having a plurality of light receiving elements for receiving light and having the plurality of light receiving elements arranged in one line.

Light source equipment 29 and light receiving unit 33 are arranged at positions facing each other over linear body 11.

The plurality of light receiving elements of light receiving unit 33 are arranged in one line along the direction of the height direction of the curvature of linear body 11, in other words, along the direction in which the top of the curvature of linear body 11 is moved when the compressive force or tensile force in the longitudinal axis direction is exerted to linear body 11.

Except for the part where illumination from light source equipment 29 is interrupted by linear body 11, a plurality of light receiving elements among the light receiving elements arranged in one line at light receiving unit 33 receive the illumination and converts it into an electric signal.

Line sensor 30 is connected to measurement instrument control unit 10 of measurement instrument 101 through an interface unit 9. Measurement instrument control unit 10 specifies the position of linear body 11, assuming that the part which does not receive illumination among the light receiving elements of light receiving unit 33 of line sensor 30 is interrupted by the curved part of linear body 11.

Moreover, measurement instrument control unit 10 is provided with a table memory 110 retaining a preset position of linear body 11 and a correlation with the compressive force or tensile force as table values.

The specified position of linear body 11 is converted by the table values of table memory 110 and becomes an output signal indicating the compressive force or tensile force.

Measurement instrument control unit 10 is connected to sensor output indicator 31.

Based on the detected position of linear body 11, measurement instrument control unit 10 converts the compressive force or tensile force into an output signal with use of the table values of table memory 110 and outputs the same as a detected value to sensor output indicator 31.

Sensor output indicator 31 visualizes the output signal into values or the like and indicates it on a display to call an attention.

Further, in the embodiment of Fig. 4, measurement instrument control unit 10, line sensor 30, and force detector 109 are connected to a calibration control unit 40 of calibration device 1 to automate the calibration operation.

It should be noted that, as will be described later, when the calibration operation is performed manually, measurement instrument 101 and calibration device 1 are not electrically connected. Sensor output indicator 31 and force detection indicator 32 directly connected to force detector 109 are visually compared, and the calibration is performed with use of a calibration operation device 35, as shown in Fig. 1.

On the other hand, when the calibration operation is performed automatically, measurement instrument 101 and calibration device 1 are electrically connected. Calibration control unit 40 includes a memory unit 41, which retains data and can read and write the data, and a CPU 42, which reads data from memory unit 41 and performs comparison and calculation.

The measured compressive force or tensile force is outputted from measurement instrument control unit 10 to electrically connected calibration control unit 40, and the calibration is performed.

At this time, for use in the comparison and calculation, calibration control unit 40 reads data stored in advance in memory unit 41 or the like of a correlation between a position of linear body 11 and the compressive force or tensile force from table memory 110 of measurement instrument control unit 10.

In calibration control unit 40, comparison with a set reference value, which will be described later, and calculation are performed, and the result of comparison and calculation are used as data for calibration.

The data used for calibration may be transmitted from calibration control unit 40 to table memory 110 of measurement instrument control unit 10 and overwritten, and used in the automated calibration operation.

Further, the data used for calibration may be outputted to force detection indicator 32 and visualized as a force exerted to force detector 109.

As described above, based on the calibrated predetermined correlation between the curvature of linear body 11 and the compressive force or tensile force exerted to linear body 11, measurement instrument control unit 10 converts the curvature of linear body 11 into the compressive force or tensile force exerted to linear body 11 and outputs the same as a measurement signal.

Optical elements such as a lens, a slit, and a filter for interrupting outdoor light may be provided in the optical system of line sensor 30 to suitably form an image of linear body 11 on line sensor 30.

An operation unit 34 such as a keyboard, a mouse, or a switch is connected to calibration control unit 40, so that the operation along the calibration order can be performed by input operation from operation unit 34.

### [Configuration of Push/Pull Movable Part]

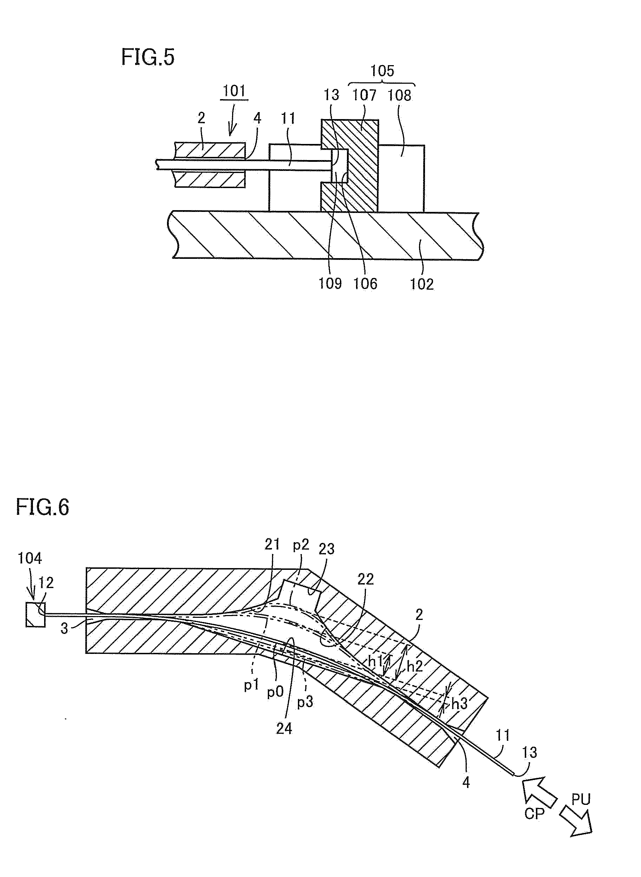

Fig. 5 represents a cross section at a position along the V-V line of Fig. 1.

Push/pull movable part 105 fixed to base 102 has a movable part 107 and a pair of movable guide parts 108, 108. The movable guide parts 108 guide movable part 107 in a slidable manner. Other end entrance 4 is arranged on a movable extension line.

An end surface of front side end 13 of linear body 11 protruding from other end entrance 4 of sensor main body 2 comes in contact with movable part 107. Linear body 11 connected by this contact can be pushed or pulled by movable part 107 in the insertion/withdrawal direction.

A pressure-receiving recessed part 106 is formed in movable part 107. Pressure-receiving recessed part 106 allows the end surface of front side end 13 of linear body 11 to come in contact and receives the same.

A load sensor as force detector 109 is provided in this pressure-receiving recess part 106.

This load sensor measures the compressive force or tensile force in the longitudinal axis direction of linear body 11 exerted from front side end 13 to force detector 109.

The measured value is converted into an electric signal and transmitted to calibration control unit 40 shown in Fig. 4.

### [Operation of Measurement Instrument]

Fig. 6 represents a part which is the same as the cross-sectional view of Fig. 3, and represents a state where the curvature of linear body 11 is changed by the compressive force or tensile force exerted in the longitudinal axis direction of linear body 11 inserted to 'sensor main body 2.

Firstly, one end side end 12 of linear body 11 protruding from one end side entrance 3 by insertion is fixed to base 102 shown in Fig. 1 by stopper part 104.

Sensor main body 2 is detachably fixed to base 102 through measurement instrument fixing part 103. Therefore, a compressive force CP or a tensile force PU exerted from front side end 13 of linear body 11 by push/pull movable part 105 causes linear body 11 to change the curvature and displace in the inward and outward directions.

Fig. 6 represents a state where, in the cross-sectional view of Fig. 2, compressive force CP or tensile force PU is exerted to linear body 11, and linear body 11 is curved in sensor main body 2.

When compressive force CP in the longitudinal axis direction is exerted to linear body 11, the curvature of linear body 11 increases. As the curvature of linear body 11 increases, the height of the curvature becomes greater.

The state of linear body 11 in which compressive force CP and tensile force PU are not exerted to linear body 11 is indicated by p0. In the unloaded state, at reference position p0, linear body 11 is curved to form an arc along the V-shaped bending at the center part of sensor main body 2 in the longitudinal direction.

When compressive force CP is exerted to linear body 11, linear body 11 is further curved from reference position p0, and the height of the curvature increases by h1 (curved position p1) as compared to reference position p0.

When greater compressive force CP as compared to curved position p1 is exerted to linear body 11, linear body 11 is further curved than curved position p1, and the height of the curvature increases as compared to curved position p1 by h2 (h2>h1) (curved position p2) as compared to reference position p0.

Moreover, as shown in Fig. 6, when tensile force PU in the longitudinal axis direction is exerted to linear body 11, the curvature of linear body 11 decreases. As the curvature of linear body 11 decreases, the height of the mountain of curvature decreases.

When tensile force PU is exerted to linear body 11 with respect to unloaded reference position p0, the curvature of linear body 11 decreases as compared to reference position p0, and the height of the curvature decreases by h3 as compared to reference position p0 (curved position p3).

### [Calibration Operation Using Indicator]

Referring back to Fig. 1, the calibration may be performed by comparing a value indicated by sensor output indicator 31 and a value indicated by force detection indicator 32 and adjusting the table values retained in table memory 110 of measurement instrument 101.

The calibration of measurement instrument 101 is performed by a manual operation. However, in this case, adjusting means provided at measurement instrument 101 for performing calibration is not particularly limited.

For example, the table values retained in table memory 110 may be adjusted with use of operating device (Fig. 1). Moreover, the position of line sensor 30 on sensor main body 2 may be adjusted.

### [Calibration Operation with Use of Calibration Control Unit]

When the curvature of linear body 11 increases as compared to the curvature of linear body 11 in the case where compressive force CP and tensile force PU are not exerted to linear body 11, measurement instrument control unit 10 converts the detected curvature into a compressive force exerted to linear body 11.

In CPU 42, the force in the insertion/withdrawal direction measured by force detector 109 and the position of linear body 11 detected by line sensor 30 are compared, and the predetermined retained in memory unit 41 is calibrated.

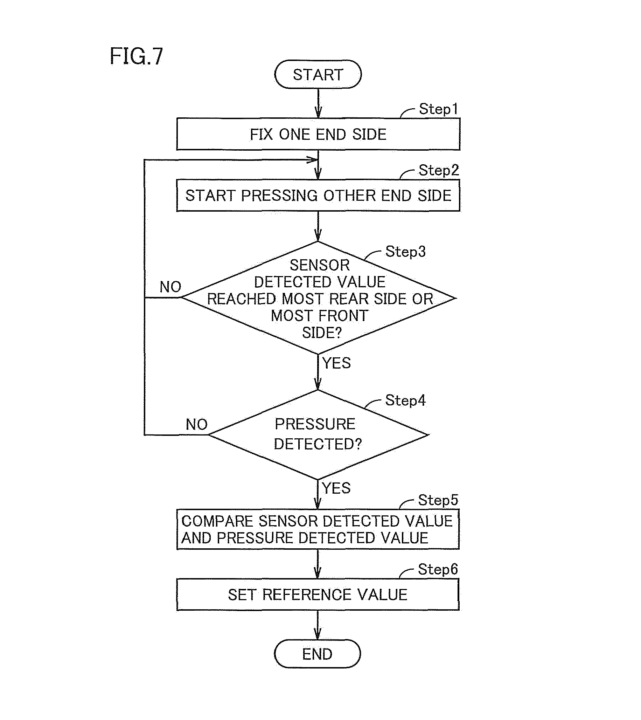

Fig. 7 is a flowchart for explanation of the calibration for calibration device 1 of measurement instrument 101 in accordance with the first embodiment.

On one side surface of base 102, a part of sensor main body 2 at the center part in the longitudinal direction is fixed through measurement instrument fixing part 103.

By fixing sensor main body 2 to the one side surface of base 102, one end side end 12 of linear body 11 is fixed in the state of being oriented in the direction of stopper part 104, and front side end 13 is fixed in the state of being oriented toward push/pull movable part 105. Then, the calibration operation is started.

In Step 1, one end side end 12 of protruding linear body 11 is fixed to stopper part 104, so that linear body 11 is fixed respect to base 102 so that it cannot be moved.

In Step 2, front side end 13 is coupled to movable part 107 of push/pull movable part 105. In other words, front side end 13 of linear body 11 abuts force detector 109 provided at pressure-receiving recessed part 106, so that the compressive force or tensile force exerted to linear part 11 can be measured based on the movement of movable part 107.

When the measurement is started while exerting compressive force CP to movable part 107, the curvature of linear body 11 in detection part 6 increases in Step 3.

In Fig. 6, at curved position p2 indicated by the two-dotted chain line, line sensor 30 detects the state where linear body 11 is stopped by abutting to the rearmost inner walls 21, 22 of detection part 6.

When it is detected that linear body 11 is in the state of being stopped at the detection position of line sensor 30, the process proceeds to next Step 4. When linear body 11 is not reached to inner wall 21, 22 yet, the process returns to Step 2 and the pressing is continued.

In Step 4, compressive force CP up to the position with a maximum curved state is detected.

Moreover, in Step 3, tensile force PU may be applied to front side end 13 until linear body 11 abuts inner wall 24 at curved position p3 indicated by the two-dotted chain line in Fig. 6 so that the curvature of linear body 11 in detection part 6 is reduced.

In this case, when line sensor 30 detects that linear body 11 has reached to the most front end, the process proceeds to next Step 4. When linear body 11 has not reached, the process returns to Step 2, and tensile force PU may be applied continuously.

In Step 4, tensile force PU is detected by force detector 109 until the position where the curved state becomes minimum is reached.

In Step 5, calibration control unit 40 compares the sensor detection value detected by line sensor 30 and compressive force CP or tensile force PU given by force detector 109.

In Step 6, the data of the reference values of table values retained in table memory 110 is calibrated with the comparison value.

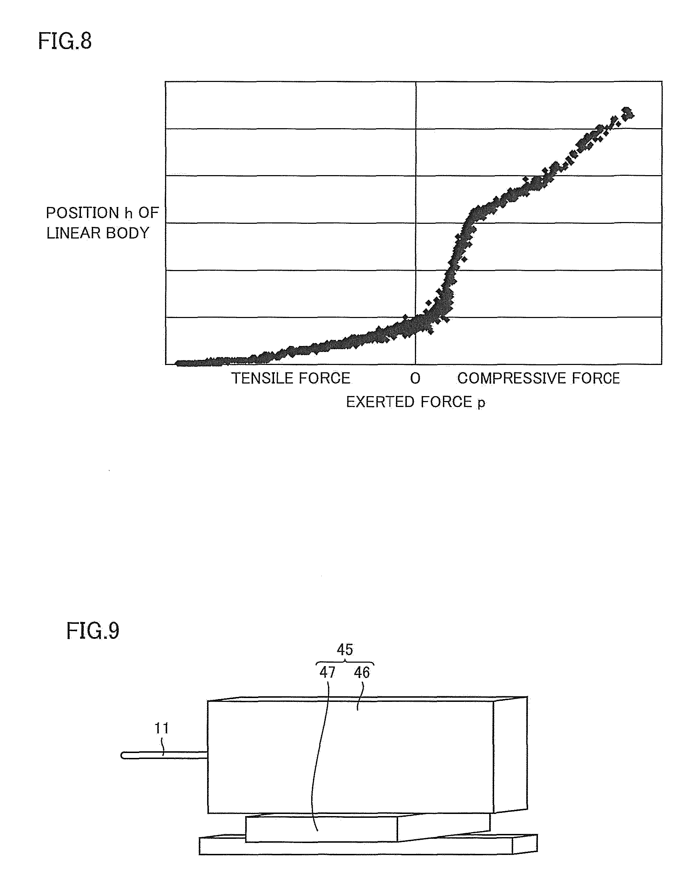

For example, the table value indicating the correlation retained in advance in table memory 110 may be adjusted. In this case, for example, position h of linear body 11, where compressive force CP and tensile force PU are balanced and applied force described later in Fig. 8 becomes zero (p=0)may be set as a reference value. Position h of linear body having an upper limit value and a lower limit value in the detectable range of detection part 6 may be set as a reference value.

Alternatively, calibration control unit 40 can perform automatically by using data calibrated by calibration control unit 40 and overwriting the table values retained in table memory 110.

Fig. 8 is a graph plotting measured values of positions obtained as a result of the displacement of linear body 11 in the state where compressive force CP and tensile force PU exerted applied force are exerted to linear body 11.

As can be seen in the plotted graph, position h of linear body 11 indicates different characteristics in the tensile force and compressive force, and is also different in accordance with a kind of linear body 11.

The data of the correlation retained in advance in table memory 110 or memory unit 41 may be set based on the graph values measured and plotted in such a manner. With use of this comparison value, the data of the correlation can be calibrated.

Accordingly, the actual assembled state and errors are incorporated, and the correlation close to the characteristic of linear body 11 can be achieved.

Therefore, this calibration device 1 of measurement instrument 101 performs calibration of measurement instrument 101 directly after assembling measurement instrument 101 without being limited to the use environment, so that the highly accurate measurement can be performed in a simple manner.

In other words, after the calibration is completed, line sensor 30 detects a position of the curved part of linear body 11 inside detection part 6.

When linear body 11 is displaced from reference position p0 of linear body 11, where the compressive force or the tensile force is not exerted to linear body 11, to the outer circumference side, measurement instrument control unit 10 converts the amount of displacement from reference position p0 to the compressive force exerted to linear body 11. When linear body 11 is displaced from the reference position to the inner circumference side, measurement instrument control unit 10 converts the amount of displacement from the reference position to the tensile force exerted to linear body 11.

Light receiving unit 33 receives the light emitted from light source equipment 29 arranged at a position opposite to light receiving unit 33 over detection part 6.

At this time, when linear body 11 is located on a light receiving element among the plurality of light receiving elements in light receiving unit 33, the light emitted by light source equipment 29 is interrupted by linear body 11.

Accordingly, the quantity of light received by the light receiving element is reduced. Then, measurement instrument control unit 10 detects the position of the light receiving element which received smaller quantity of light, and specifies the position of linear body 11.

Thus, the height of the curvature is displaced in accordance with the curvature of linear body 11, and line sensor 30 is interrupted, so that the position of linear body 11 is calculated from the part at which the shadow of the illumination is detected.

Based on the data which contains accurate correlation data registered again in memory unit 41 by the calibration of the reference value as shown in Fig. 8, for example, based on the predetermined correlation between the height of the curvature of linear body 11 detected by line sensor 30 and the compressive force or tensile force exerted to linear body 11, measurement instrument control unit 10 converts the height of the curvature of linear body 11 into the compressive force or tensile force exerted to linear body 11 and outputs the same.

In such a manner, the compressive force or tensile force exerted in the longitudinal axis direction of linear body 11 can be measured accurately.

As described above, in the state where line sensor 30 is assembled to detection part 6 of sensor main body 2, push/pull movable part 105 which allows linear body 11 to be pushed and withdrawn in the insertion/withdrawal direction and stopper part 104 fixing the linear body 11 are fixed to sensor main body 2 so that the relative position cannot be changed.

Therefore, reusable line sensor 30 is assembled to new sensor main body 2 replaced in an operation room or the like, and force detector 109 detects the force in the insertion/withdrawal direction exerted to linear body 11.

Calibration control unit 40 compares the detected force in the insertion/withdrawal direction is compared with the position of linear body 11 detected by line sensor 30, and the calculated result is outputted to measurement instrument control unit 10, and the calibration is performed.

Further, with the calibration of the reference value shown in Fig. 8 retained in advance by memory unit 41, an accurate reference value suitable for a kind of linear body 11 can be calibrated.

Therefore, the calibration of measurement instrument 101 can be performed in a simple manner without the limitation by the use environment. Consequently, the highly accurate measurement by measurement instrument 101 can be performed with use of replaced new and clean sensor main body 2.

### [Modified Example 1]

Fig. 9 is a perspective view representing a push/pull movable part of Modified Example 1 in the first embodiment. In the following description, the parts which are the same as the first embodiment have the same reference numerals allotted. The names and functions of those are also the same. Thus, detailed description thereof will not be repeated.

In this Modified Example 1, the push/pull movable part is constituted of a linear guide mechanism 45 in place of push/pull movable part 105 of the first embodiment.

Linear guide mechanism 45 is a linear motion guide mechanism having a movable part 46 which is slidable on a straight line along a linear guide 47. An end of linear body 11 is coupled to moving part 46.

Since other configuration and effect are the same as the first embodiment, description thereof will not be repeated.

### [Modified Example 2]

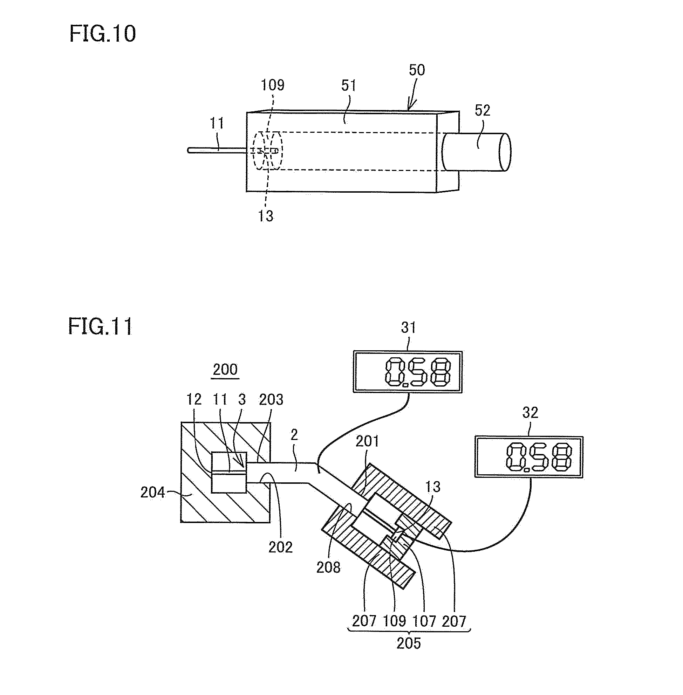

Fig. 10 is a perspective view representing a push/pull movable part 50 of Modified Example 2 of the first embodiment.

Push/pull movable part 50 is a linear motion guide mechanism having a cylindrical movable part 52 which is slidable along a movable guide cylinder 51.

Force detector 109 is fixed to an end face of movable part 52 on front side end 13 of linear body 11.

Since other configuration and effect are the same as the first embodiment, description thereof will not be repeated.

### [Second Embodiment]

Fig. 11 is a plan view representing a calibration device 200 of a measurement instrument of the second embodiment. In the following description, the parts which are the same as the first embodiment have the same reference numerals allotted. The names and functions of those are also the same. Thus, detailed description thereof will not be repeated.

In the second embodiment, a stopper part 204 is used in place of stopper part 104 of the first embodiment, and a push/pull movable part 205 is used in place of push/pull movable part 105.

At the outer side part of sensor main body 2 of the second embodiment, a housing is integrally provided, and this housing part serves as base 102 which is fixing means of the first embodiment.

Stopper part 204, at an outer side surface 203 near the one end side of the housing formed integrally with this sensor main body 2, has a fixing opening 202 fitted to this outer side surface 203 from the outer side and fixed therein.

Moreover, push/pull movable part 205 is fixed to sensor main body 2 by fitting a fixing opening part 208 formed integrally with movable guide part 207 to an outer side surface 201 near entrance 4 of the housing at the other end.

Therefore, in addition to the effect of the first embodiment, base 102 is not required, thus the number of parts can be reduced. Moreover, in place of base 102, the housing of sensor main body 2 is used as fixing means. Therefore, as compared to calibration device 1 of the first embodiment, the external dimension of calibration device 200 can be further reduced.

Since other configuration and effect are the same as the first embodiment, description thereof will not be repeated.

### [Third Embodiment]

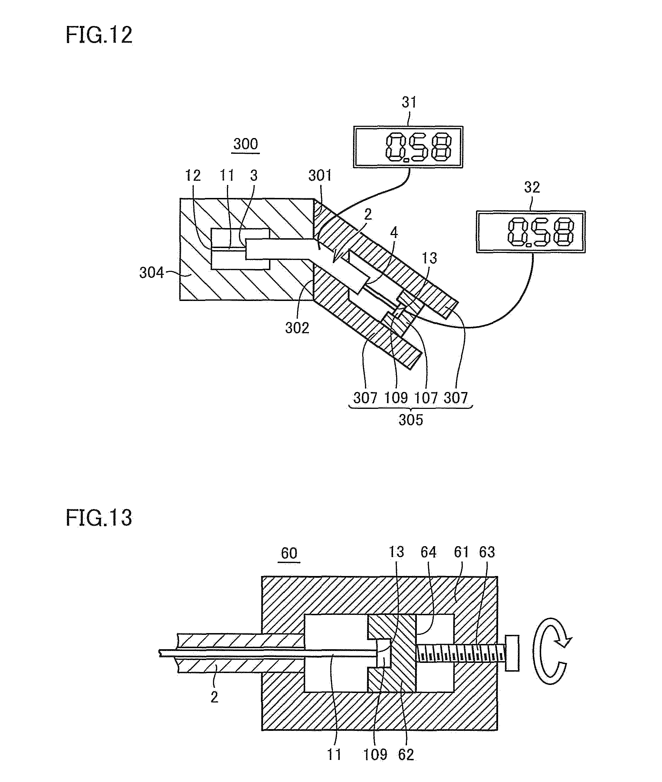

Fig. 12 is a plan view representing a configuration of a calibration device 300 for a measurement instrument of the third embodiment. In the following description, the parts which are the same as those of the first and second embodiments have the same reference numerals allotted. The names and functions of those are also the same. Thus, detailed description thereof will not be repeated.

In the third embodiment, in place of base 102 of the first embodiment, fixing means is mainly constituted of stopper part 304 and push/pull movable part 305 provided in the periphery of sensor main body 2.

In other words, stopper part 304 is attached to one end side entrance 3 of sensor main body 2.

Moreover, push/pull movable part 305 is attached to other end entrance 4.

Then, the housing of sensor main body 2 is sandwiched from opposite sides, so that adjoining face part 301 of stopper part 304 and adjoining face part 302 of push/pull movable part 305 are adjoined and fixed integrally on the outer side of sensor main body 2.

On the outer side of sensor main body 2 of the third embodiment, stopper part 304 and push/pull movable part 305 are integrally formed and serve as base 102 which is fixing means of the first embodiment.

Therefore, in addition to the effect of the first embodiment, base 102 is not required, and the number of parts can be reduced, so that calibration device 300 can be further reduced in size as compared to calibration device 1 of the first embodiment and calibration device 200 of the second embodiment.

Additionally, as with calibration device 200 of the second embodiment, it would not necessary to provide an additional part for fixing stopper part 204 and push/pull movable part 205 to the housing of sensor main body 2. Therefore, increase in the number of parts can be suppressed.

Since other configuration and effect are the same as the first and second embodiments, description thereof will not be repeated.

### [Modified Example 3]

Fig. 13 is a cross-sectional view at a position along the axial direction representing push/pull movable part 60 of the second and third embodiments. In the following description, the parts which are the same as the second and third embodiments have the same reference numerals allotted. The names and functions are also the same. Thus, detailed description thereof will not be repeated.

Push/pull movable part 60 is guided slidably by movable guide part 61, and has a movable top member 62 in contact with front side end 13 of linear body 11 and a screw member 63 capable of adjusting the position of movable top member 62 from the a back side 64.

In push/pull movable part 60 described in Modified Example 3 which is configured in such a manner, in addition to the effect of the first and second embodiments, screw member 63 is rotated and screwed, so that the position in the axial direction can be changed. When the position of movable top member 62 is changed, the compressive force or tensile force given to linear body 11 can be adjusted.

In this case, being different from the case of pushing with a human hand, a constant force can be exerted for a certain period of time.

Since other configuration and effect are the same as the second and third embodiments, the description thereof will not be repeated.

### [Modified Example 4]

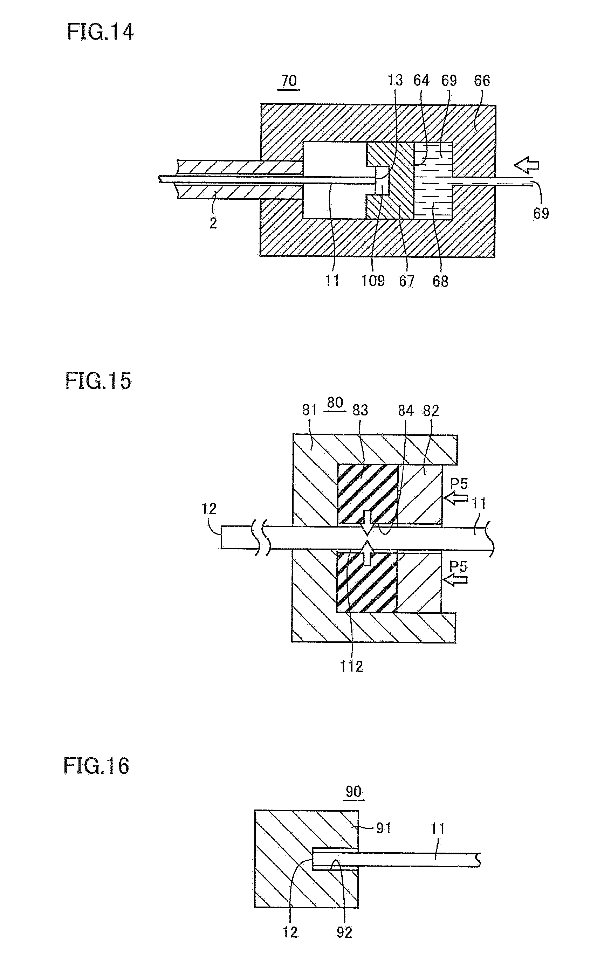

Fig. 14 is a cross-sectional view representing push/pull movable part 70 of Modified Example 4 at a position along the axial direction in the second and third embodiments. In the following description, the parts which are the same as the first embodiment and calibration device 200 of Modified Example 3 have the same reference numerals allotted. Names and functions of those are also the same. Thus, detailed description thereof will not be repeated.

In this Modified Example 4, a back pressure chamber 68 is formed between a back face 64 of a movable top member 67 and an inner side surface of movable guide part 66, and liquid 69 flowing in and out through a water passage can change the position of movable top member 67.

Therefore, the position of movable top member 67 can be adjusted by changing the pressure of liquid 69, so that the compressive force or tensile force exerted to linear body 11 can be changed.

Also in this case, as with the case of using screw member 63 of Fig. 13, a constant force can be exerted for a certain period of time.

Since other configuration and effect are the same as the second and third embodiments, description thereof will not be repeated.

### [Modified Example 5]

Fig. 15 is a cross-sectional view representing stopper part 80 of Modified Example 5 used in the first to third embodiments at a position along the axial direction. In the following description, the parts which are the same as the first and second embodiments and Modified Example 3 have the same reference numerals allotted. Names and functions of those are also the same. Thus, detailed description thereof will not be repeated.

Stopper part 80 includes an elastic member 83 made of elastically deformable rubber formed to have a disk-like shape with a size in a predetermined thickness direction. A fitting hole 84 is formed at a center part of elastic member 83, and one end side end 12 of linear body 11 is inserted.

In this state, using a cylindrical-shaped syringe 81 and a plunger 82 as a press-fitting fixing member, elastic member 83 is pressed toward the inner surface of syringe 81. The plunger 82 is provided slidably along linear body 11.

Moreover, in the second and third embodiments, when stopper part 80 is mounted to a part near one end side entrance 3 of sensor main body 2, an end of sensor main body 2 comes into contact with plunger 82 and pushes with pressure P5, P5 generated by pushing.

When the pushing force is given to elastic member 83 as pressure P5, P5, the elastic member is deformed toward the inner side in the radial direction as indicated by a white arrow. Accordingly, the inner surface of fitting hole 84 can be pressed onto a peripheral surface 112 of linear body 11.

Since other configuration and effect are the same as the first to third embodiments, description will not be repeated.

### [Modified Example 6]

Fig. 16 is a cross-sectional view representing a stopper part 90 of Modified Example 6 used in the first to third embodiments at a position along the axial direction. In the following description, the parts which are the same as the first and second embodiments have the same reference numerals allotted. Names and functions of those are also the same. Thus, detailed description thereof will not be repeated.

At a stopper member 91 constituting stopper part 90, a recessed receiving part 92 provided as a recess having a certain size in the depth direction is formed. One end side end 12 of linear body 11 is inserted and fitted to recessed receiving part 92.

Since other configuration and effect are the same as the first to third embodiments, description thereof will not be repeated.

### [Modified Example 7]

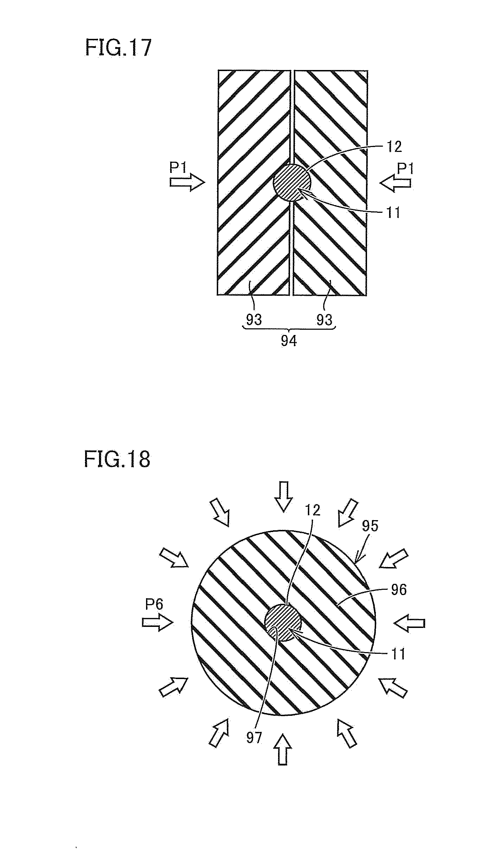

Fig. 17 is a cross-sectional view representing stopper part 93 used in the first to third embodiments at a position along the vertical direction. In the following description, the parts which are the same as the first to third embodiments have the same reference numerals allotted. Names and functions of those are also the same. Thus, description thereof will not be repeated.

A clamping and fixing member 94 of Modified Example 7 clamps and fixes one end side end 12 of linear body 11 between a pair of elastic members 93, 93.

Since other configuration and effect are the same as the first to third embodiments, description thereof will not be repeated.

### [Modified Example 8]

Fig. 18 is a cross-sectional view representing stopper part 95 of Modified Example 8 used in the first to third embodiments at a position along the vertical direction. In the following description, the parts which are the same as the first to third embodiments have the same reference numerals allotted. Names and functions of those are also the same. Thus, detailed description there of will not be repeated.

Stopper part 95 is fitted by inserting one end side end 12 of linear body 11 into fitting hole 97 formed at a radially central part of deformable columnar elastic member 96.

Since other configuration and effect are the same as the first to third embodiments, description thereof will not be repeated.

### [Fourth Embodiment]

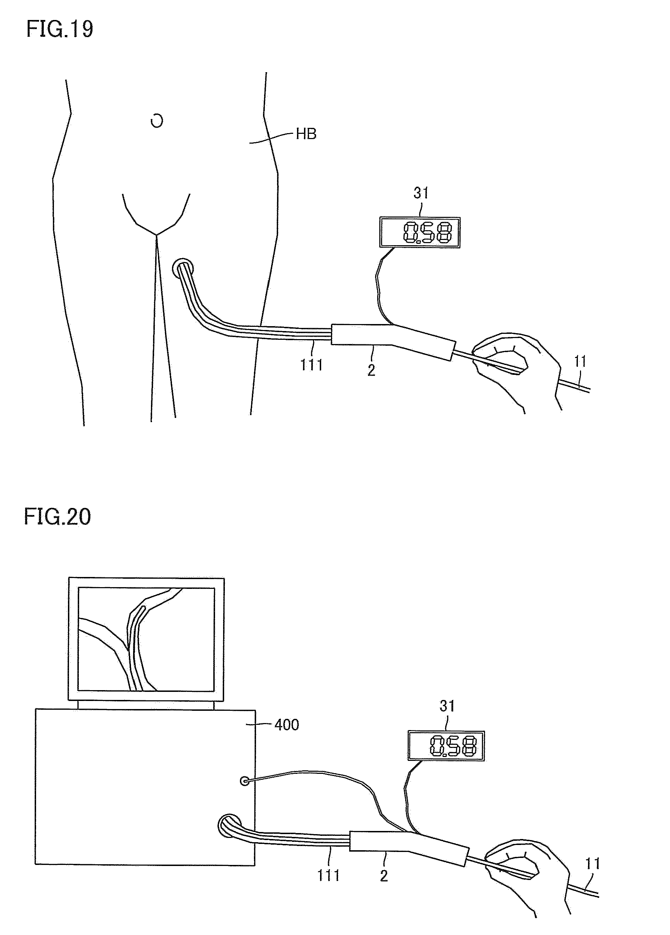

Fig. 19 represents a state where sensor main body 2 of the calibrated measurement instrument in the fourth embodiment is used for embolism treatment of aneurysm of a human body HB.

Sensor main body 2 can be readily calibrated by using the calibration device 1 of the present invention described in the above-mentioned first to third embodiments even in a place where a strict management is required in view of the sanitization in the use environment such as an operating room.

Moreover, a sensor output indicator 31 is connected to sensor main body 2 leading out a catheter 111. Then, the compressive force or tensile force exerted to linear body 11 are visualized and indicated by sensor output indicator 31.

Since other configuration and effect are the same as the first to third embodiments, description will not be repeated.

### [Fifth Embodiment]

Fig. 20 represents a state where sensor main body 2 of the measurement instrument calibrated in the fifth embodiment is used for a training simulation machine 400.

Sensor main body 2 can be readily calibrated with use of calibration device 1 of the present invention described in the first to third embodiments before the use in simulation device 400. Linear body 11 such as a wire member is inserted to sensor main body 2 of measurement instrument 101 after the calibration.

Moreover, sensor output indicator 31 is connected to sensor main body 2 to which catheter 111 is mounted and from which linear body 11 is lead out from inside.

Then, the compressive force or the tensile force exerted to linear body 11 are visualized and indicated by sensor output indicator 31.

When the compressive force or tensile force is given to linear body 11 from simulation device 400, the compressive force or tensile force exerted to linear body 11 in the longitudinal axis direction is measured by line sensor 30 mounted in sensor main body 2 of measurement instrument 101.

The actual measured value given from line sensor 30 is accurate since the calibration has already been completed.

Moreover, the feeling given to an operator who operates linear body with the compressive force or tensile force exerted to linear body 11 in the longitudinal axis direction is set to be close to the actual state, so that the training with use of simulation device 400 becomes close to the actual treatment.

It should be noted that, in place of force detection indicator 32, or together with force detection indicator 32, a speaker or a lamp which can output a caution sound or caution light may be connected to sensor main body 2.

When the compressive force or tensile force exerted to linear body 11 exceeds a predetermined value, the caution sound or caution light is outputted from the speaker or lamp.

Since other configuration and effect are the same as the first to third embodiments, description thereof will not be repeated.

Finally, the embodiments described above will be summarized with reference the drawings again.

According to the present invention, sensor main body 2 provided with one end side entrance 3 and the other end side entrance 4 through which linear body 11 having a flexibility is inserted and withdrawn, detection part 6 which allows displacement of linear body 11 communicating with sensor main body 2, and line sensor 30 detachably provided at detection part 6 and detecting the displacement of linear body 11 at the position of linear body 11 are provided.

Measurement instrument control unit 10 measures the compressive force or tensile force exerted in the longitudinal axis direction of linear body 11 from the predetermined correlation based on the position of linear body 11 detected by line sensor 30.

Stopper part 104 fixes linear body 11 near one end side entrance 3. Moreover, push/pull movable part 105 allows linear body 11 to be pushed and pulled in the insertion/withdrawal direction near the other end side entrance 4, and fixes linear body 11 so that the relative position to sensor main body 2 cannot be changed.

Force detector 109 is interposed between push/pull movable part 105 and linear body 11, and measures the force exerted in the insertion/withdrawal direction from push/pull movable part 105 to linear body 11. The force in the insertion/withdrawal direction measured by force detector 109 and the position of linear body 11 detected by line sensor 30 are compared, and the predetermined correlation is calibrated.

In the calibration device of the measurement instrument of the present invention configured in such a manner, sensor main body 2 having line sensor 30 assembled to detection part 6 is fixed immovably to push/pull movable part 105 which allows linear body 11 to be pushed and pulled in the insertion/withdrawal direction and fixing stopper part 104.

Therefore, even in the place such as an operating room where a strict management is required for the use environment in view of the sanitation, reusable line sensor 30 is assembled to detection part 6 of sensor main body 2, and can be mounted integrally to calibration device 1 so as to readily make it possible to be immovable.

When the force in the insertion/withdrawal direction exerted to the linear body is detected by the force detector 109, it is compared with the position of linear body 11 detected by line sensor 30, and the calibration is performed.

Therefore, the calibration of the measurement instrument can be performed in a simple manner without limitation by the use environment, and the highly accurate measurement by the measurement instrument can be performed.

Moreover, in the first to third embodiments, the item as linear body 11 used for the actual treatment is inserted to sensor main body 2 and the calibration is performed. However, not limited to this, for example, it may be an item which is the same kind as linear body 11 used for the actual treatment and has a displacement characteristics inward and outward in the curving direction when a force in the insertion/withdrawal direction is similarly exerted.

It should be noted that, in the first embodiment, it is configured that light receiving unit 33 of line sensor 30 is arranged at a position facing light source equipment 29 and that light receiving unit 33 receives illumination.

However, not limited to this, light source equipment 29 and light receiving unit 33 may be arranged in line, and a reflector such as a mirror reflecting light emitted from light source equipment 29 may be arranged at a position facing light source equipment 29.

In this case, the reflected light reflected by the reflector among light emitted from light source equipment 29 is received by light receiving unit 33, so that the curvature of linear body 11 can be detected in the same manner.

Moreover, in place of the one-dimensional array sensor like line sensor 30, the detection of the curvature of linear body 11 can be performed also with use of a two-dimensional array sensor having a plurality of light receiving element arranged in line on the flat plane.

Further, the position sensor is all necessary to detect the curvature of linear body 11. Therefore, it is not limited to the optical sensor such as line sensor 30.

For example, a contactless distance sensor detecting a height of curvature or a position sensor detecting the displacement from the reference position of linear body 11 can be used as the position sensor.

In the first embodiment, description was made such that sensor output indicator 31 shown in Fig. 1 and force detection indicator 32 are compared to perform manual or automatic calibration. However, not limited to this, calibration control unit 40 capable of performing the automatic calibration may be omitted.

In this case, as shown in Fig. 1, the indication by the sensor output indicator 31 and the indication by force detection indicator 32 connected directly to force detector 109 are compared visually.

Then, an adjustment knob for calibration provided in advance at measurement instrument 101 or sensor output indicator 31, or operating device 35 for calibration connected from outside allows the indicated value of sensor output indicator 31 and the indicated value of force detection indicator 32 to be matched, so that the calibration can be performed by manual operation.

Moreover, for example, not limited to the calibration performed manually or automatically as shown in the first embodiment, such as the automatic calibration by calibration control unit 40, it would be all necessary that force detector 109 which measures a force in the insertion/withdrawal direction exerted from push/pull movable part 105 to linear body 11 is provided so as to allow calibration with any one of manual operation and automatic operation, and that the calibration is performed by comparison of the detected values.

Moreover, for example, the calibration may be performed including the adjustment by the manual operation such as the one making the calibration operation be partially automatic or semi-automatic, and a plurality of calibration operations may be combined.

It should be understood that the embodiments disclosed herein are illustrative and non-restrictive in every respect. The scope of the present invention is defined not by the description above but by the scope of claims, and is intended to include any modifications within the scope and meaning equivalent to the scope of claims.

### REFERENCE SIGN LIST

1, 200, 300 calibration device; 2 sensor main body; 3 one end side entrance; 4 other end side entrance; 5 confining part; 6 detection part; 9 interface unit; 10 measurement instrument control unit; 11 linear body; 12 one end; 13 front side end; 21 inner wall; 22 inner wall; 23 recess; 24 inner wall; 29 light source equipment; 30 line sensor; 31 sensor output indicator; 32 force detection indicator; 33 light receiving unit; 40 calibration control unit; 41 memory unit; 42 CPU; 43 control unit; 45 linear guide mechanism; 46 movable part; 47 linear guide; 50 push/pull movable part; 51 movable guide cylinder; 52 movable part; 59 screw member; 60 push/pull movable part; 61 movable guide part; 62 movable top member; 63 screw member; 64 back face; 66 movable guide part; 67 movable top member; 68 back pressure chamber; 69 fluid; 70 push/pull movable part; 71 fixing means; 72 fixing tool; 73, 73 elastic body; 76 elastic body; 80 fixing mechanism; 81 syringe; 82 plunger; 83 elastic body; 84 insertion fixing hole; 101 measurement instrument; 102 base; 103 measurement instrument fixing part; 90, 93, 95, 104, 204, 304 stopper part; 105 push/pull movable part; 106 pressure-receiving recess part; 107 moving part; 108 movable guide part; 109 force detector; 111 catheter; 112 outer peripheral surface; 201, 203 outer peripheral surface; 202, 208 fixing opening part; 400 simulation device; CP compressive force; PU tensile force.

## Claims

1. A calibration device for a measurement instrument, said measurement instrument including:
a sensor main body provided with a one end side entrance and an other end side entrance which a linear body having a flexibility is inserted to and withdraw form;
a detection part allowing a displacement of said linear body which is in communication with said sensor main body; and
a position sensor detachably provided in said detection part and configured to detect a position of said linear body,
said measurement device measuring a compressive force or a tensile force exerted in a longitudinal axis direction of said linear body using a predetermined correlation based on a position of a linear body detected by said position sensor,
said calibration device comprising:
a stopper part for fixing one end side of said linear body at said one end side entrance;
a push/pull movable part configured to locate a front side on a side opposite to said one end side and to be pushed and pulled in an insertion/withdrawal direction at said other end side entrance;
fixing means which fixes said stopper part and said push/pull movable part so that relative positions thereof with respect to said sensor main body cannot be changed; and
a force detector interposed between said push/pull movable part and said linear body and configured to measure a force in an insertion/withdrawal direction exerted from said push/pull movable part to said linear body.

2. The calibration device for a measurement instrument according to claim 1, wherein
said fixing means includes a base, and
said base is provided with a measurement instrument fixing part for fixing said sensor main body on a plane on which said push/pull movable part and said stopper part are fixed, and
said sensor main body, said push/pull movable part, and said stopper part are arranged at desired positions on said base.

3. The calibration device for a measurement instrument according to claim 1, wherein
said fixing means includes a housing formed integrally with said sensor main body, and
said stopper part is mounted and fixed near a one end side entrance of said housing, and
said push/pull movable part is mounted and fixed near an other end side entrance of said housing.

4. The calibration device for a measurement instrument according to claim 1, wherein
by fixing said stopper part to said one end side entrance, and fixing said push/pull movable part to said other end side entrance, said fixing means is configured to adjoin said stopper part and said push/pull movable part and to fix said stopper part and said push/pull movable part integrally with said sensor main body so as to clamp said sensor main body.

5. The calibration device for a measurement instrument according to any one of claims 1 to 4, further comprising: a calibration control unit comparing a force in an insertion/withdrawal direction measured by said force detector and a position of said linear body detected by said position sensor to calibrate said predetermined correlation.

6. The calibration device for a measurement instrument according to any one of claims 1 to 5, wherein said push/pull movable part is a linear motion guide mechanism having a movable pillar part which is slidable along an inner side of a cylindrical movable guide part.

7. The calibration device for a measurement instrument according to any one of claims 1 to 6, wherein
said push/pull movable part has:
a movable top member coupling a front side of said linear body; and
a screw member configured to adjust a position of said movable top member.

8. The calibration device for a measurement instrument according to any one of claims 1 to 6, wherein
said push/pull movable part has:
a movable top member coupling a front side of said linear body; and
a back pressure chamber into which fluid used for adjusting a position of said movable top member is pressed.

9. The calibration device for a measurement instrument according to any one of claims 1 to 7, wherein
said stopper part has a press-fit fixing member, in a state where one end side of said linear body is inserted to a fitting hole formed in a deformable elastic member, the press-fit fixing member giving a pressure to said elastic member and deforming said elastic member to allow an inner surface of said fitting hole to be press-fitted to a peripheral surface of said linear body.

10. The calibration device for a measurement instrument according to any one of claims 1 to 7, wherein said stopper part has a fixing member having a recessed receiving part in which one end side of said linear body is inserted and fitted.

11. The calibration device for a measurement instrument according to any one of claims 1 to 7, wherein said stopper part has a clamping fixing member which clamps and fixes one end side of said linear body between a pair of deformable elastic members.

12. The calibration device for a measurement instrument according to any one of claims 1 to 7, wherein said stopper part has a fitting fixing member in which one end side of said linear body is inserted and fitted to a fitting hole formed in said deformable elastic member.

13. The calibration device according to any one of claims 1 to 12, wherein said calibration device is used for calibrating a measurement instrument incorporated in a medical equipment.

14. The calibration device according to any one of claims 1 to 12, wherein said calibration device is used for calibrating a measurement instrument incorporated in a training simulator.

15. A method for calibrating a measurement instrument for measuring a compressive force or a tensile force in a longitudinal axis direction exerted to a linear body having a flexibility based on a change in curvature of said linear body, said method comprising the steps of:
inserting said linear body into a cavity of a detection part provided in said measurement instrument;
detecting a degree of curvature of said linear body in said detection part;
detecting a compressive force or a tensile force in a longitudinal axis direction exerted to said linear body; and
carrying out calibration by comparing said detected compressive force or tensile force with curvature of a linear body.
